# FASCICULE DE BREVET EUROPEEN

(11) **EP 0 791 063 B1**
(45) Date de publication et mention de la délivrance du brevet: **26.01.2005**
(21) Numéro de dépôt: 95939335.6
(22) Date de dépôt: 07.11.1995
(51) Int. Cl.: C12N 15/31, C07K 14/26, A61K 39/108, A61K 39/155, A61K 39/385, A61K 31/715

(54) **PROTEINE PORTEUSE A EFFET ADJUVANT, COMPLEXE IMMUNOGENE LA CONTENANT, LEUR PROCEDE DE PREPARATION, SEQUENCE NUCLEOTIDIQUE ET VACCIN**
TRÄGERPROTEIN MIT ADJUVANT AKTIVITÄT, DIESE ENTHALTENDE IMMUNOGENE KOMPLEXE, IHRE HERSTELLUNG, NUKLEOTIDSEQUENZ UND IMPFSTOFF
CARRIER PROTEIN HAVING AN ADJUVANT EFFECT, IMMUNOGENIC COMPLEX CONTAINING SAME, PREPARATION METHOD THEREFOR, NUCLEOTIDE SEQUENCE AND VACCINE

(30) Priorité: 07.11.1994 FR 9413306
(43) Date de publication de la demande: 27.08.1997
(73) Titulaire: PIERRE FABRE MEDICAMENT, 92100 Boulogne (FR)
(72) Inventeur: BINZ, Hans, F-74160 Beaumont (FR); BAUSSANT, Thierry, F-01200 Bellegarde (FR); HAEUW, Jean-François, F-74160 Saint-Julien-en-Genevois (FR); NGUYEN NGOC, Thien, F-74160 Saint-Julien-en-Genevois (FR)
(74) Mandataire: Ahner, Francis
(86) Numéro de dépôt international: PCT/FR1995/001463
(87) Numéro de publication internationale: WO 1996/014415

(56) Documents cités:
- WO-A-89/05823
- WO-A-92/04375
- WO-A-93/14207
- WO-A-95/27787
- JOURNAL OF GENERAL MICROBIOLOGY, vol. 137, no. 8, pages 1911-1921, LAWRENCE J.G. ET AL. 'Molecular and evolutionary relationships among enteric bacteria'

## Description

La présente invention concerne des adjuvants destinés à être associés à une molécule pour améliorer son activité, en particulier pour augmenter l'intensité de la réponse immunitaire. Elle concerne également des complexes contenant un tel adjuvant associé à une molécule active.

La molécule active peut notamment être une protéine, un peptide, un polysaccharide, un oligosaccharide ou un acide nucléique, ADN ou ARN.

La mise au point de vaccins parfaitement définis et dépourvus d'effets secondaires marqués, nécessite l'emploi d'antigènes vaccinants de faible masse moléculaire, tels que des peptides ou des oligosaccharides. Ces antigènes de faible masse, mais aussi certains antigènes de masse moléculaire supérieure tels que les polysaccharides de la paroi bactérienne, ne peuvent induire seuls une réponse immunitaire durable et intense. Il est indispensable de lier ces antigènes, par voie chimique ou par génie génétique, à des protéines porteuses.

Les protéines porteuses, actuellement utilisées, sont de deux types :
- les anatoxines tétanique et diphtérique : l'emploi trop fréquent de ces protéines porteuses risque d'aller à l'encontre d'une réponse intense contre l'haptène et risque de poser des problèmes d'immunotoxicologie,
- un extrait de protéine membranaire de Neisseria meningitidis (OMPC) : est constitué par une protéine membranaire contaminée par des lipides et des LPS.

Le brevet EP- 267 204 a proposé l'utilisation d'une molécule de support destinée à être couplée à un immunogène, et consistant en une protéine de membrane d'E. coli ou de Salmonella.

Le document lawrence et al. (Journal of General Microbiology, 137,8, 1911-1921, 1992) décrit les séquences nucléotiques partielles du locas codart pour la proteine de membrare externe de type A (OmpA) de différentes espèces d'entérobactéries, dont klebsiella pneumoniae, afin de permettre d'établir les relations phylogénétiques entre ces espèces.

La Demanderesse a démontré qu'une protéine extraite de la membrane externe de Klebsiella pneumoniae permet d'améliorer considérablement la réponse immunitaire à un antigène ou un haptène lorsqu'elle est administrée en même temps que celui-ci à un hôte. Plus particulièrement, une protéine OmpA, la protéine P40 de K. pneumoniae, peut être utilisée comme adjuvant dans des complexes immunogènes, où elle est associée à un élément immunogène.

Les conjugués chimiques issus d'un couplage de peptides à la P40 donnent de bons résultats, et une évaluation de la réponse immunitaire montre des réponses en anticorps contre ces peptides supérieures à celles observées en utilisant les protéines porteuses de référence, KLH ou TT.

Toutefois, les antigènes peptidiques sont greffés de manière préférentielle sur la partie C-terminale de la séquence, partie de la molécule la plus immunogène, (Puohiniemi, R et al., *1990*, Infect Immu. 58 1691-1696. Ceci peut poser un problème sérieux pour les protéines de fusion contenant la séquence complète de P40. Ainsi, l'utilisation d'un fragment de la séquence supportant l'activité adjuvante, minimiserait davantage l'immunogénicité de la protéine porteuse et les risques liés à cette immunogénicité.

C'est pourquoi la présente invention a pour objet un complexe immunogène du type comprenant un élément immunogène, associé à un adjuvant augmentant l'intensité de la réponse immunitaire, caractérisé en ce que l'élément immunogène est un antigène ou un haptène, et en ce que l'adjuvant comprend au moins une partie de la protéine P40 de Klebsiella pneumoniae, ladite partie consistant en la séquence comprise entre les amino acides 1 à 179, 108 à 179 ou 127 à 179 da la séquence SEQ 19 N° 2.

En particulier, l'invention décrit un adjuvant constitué d'une protéine ou d'un peptide présentant la séquence de P40 substantiellement dépourvue des parties immunogènes.

Ces fragments de P40 de l'adjuvant selon l'invention sont notamment :
- la séquence de P40 dépourvue de la partie C terminale périsplasmatique immunogène,
- une séquence contenant la 3ème et la 4ème boucle extramembranaire flanquant une séquence intramembranaire.
- une séquence contenant une boucle extramembranaire invariable et la séquence intramembranaire adjacente.

On définit comme boucles extramenbranaires invariables les séquences de P40 homologues avec les séquences des boucles conservées entre différentes espèces d'entérobactéries. Les séquences des bouclcs extramenbranaires non conservées au cours de l'évolution sont dénommées boucles variables. La localisation des boucles extramembranaires est réalisée d'après le modèle de VOGEL et JAHNIG (1986, J. Mol. Biol., 190 : 191-199) concernant l'OmpA d'E. coli.

Le choix des fragments et plus particulièrement la troisième séquence (acides aminés 127 à 179) est fondé sur l'hypothèse selon laquelle les boucles extramenbranaires invariables (conservées entre les OmpA des différentes entérobactéries) contiennent des séquences reconnues par des cellules immunocompétentes, ces dernières pouvant posséder des récepteurs reconnaissant ces séquences.

La reconnaissance spécifique de ces séquences par des cellules présentatrices d'antigènes permettrait de cibler des antigénes vers ces cellules et ainsi d'induire un effet adjuvant.

C'est pourquoi, l'un des objets de l'invention est un produit adjuvant qui consiste en la séquence comprise entre les amino-acides 1 à 179 de la protéine P40 de K. pneumoniae, ou une séquence présentant au moins 80% et de préférence au moins 90% d'homologie avec la séquence comprise entre les amino-acides numérotés 1 et 179 de la séquence de la protéine P40 de K. pneumoniae de séquence SEQ ID N°. 2.

Un autre objet de l'invention est un adjuvant qui consiste en la séquence comprise entre les amino-acides 108 à 179 de la protéine P40 de K.pneumoniae ou une séquence présentant au moins 80% d'homologie et de préférence au moins 90% d'homologie avec la séquence comprise entre les amino-acides numérotés 108 et 179 de la protéine P40 de K. pneumoniae de sequence SEQ ID N° 2.

Selon un autre aspect, l'invention a pour objet un adjuvant qui consiste en la séquence comprise entre les amino-acides numérotés 127 à 179 de la protéine P40 de K. pneumoniae ou une séquence présentant au moins 80% et de préférence au moins 90% d'homologie avec la séquence comprise entre les amino-acides numérotés 127 à 179 de la protéine P40 de K. pneumoniae de séquence SEQ ID N° 2.

Les séquences ID n° 2, ID n° 4, ID n° 6 et ID n° 8 correspondent à des adjuvants selon l'invention. Cette protéine et ces peptides adjuvants peuvent notamment être préparés à partir de membranes de bactéries du genre Klebsiella pneumoniae. Le procédé comprend alors les étapes suivantes :
a) précipitation des lipopolysaccharides par addition de détergent et d'un sel de cation divalent et récupération du surnageant,
b) précipitation des protéines du surnageant et remise en suspension du culot,
c) chromatographie de la suspension sur échangeur d'anions et récupération des fractions contenant le produit adjuvant,
d) chromatographie sur échangeur de cations et récupération de la fraction contenant le produit adjuvant,
e) concentration de la fraction obtenue à l'issue de l'étape d) pour récupérer un produit adjuvant sous forme de protéine ou de peptide, essentiellement dépourvu de liposaccharides.

Des étapes de dialyse peuvent avantageusement intervenir entre, respectivement, les étapes b) et c) et les étapes c) et d).

L'invention a également pour objet les complexes immunogènes pouvant être obtenus à partir des différents adjuvants.

L'adjuvant peut être associé à l'élément immunogène par couplage chimique.

Ce couplage covalent de l'haptène peptidique à l'adjuvant peut être effectué d'une façon bien connue dans la technique. Des réactifs appropriés à cette fin comprennent notamment les esters de N-succinimide, les carbodiimides, l'EEDQ (N-éthoxycarbonyl-2-éthoxy-1,2-dihydroquinoléine) et similaires.

On peut également fusionner par génie génétique le fragment de la protéine P40 en cause et l'élément immunogène.

La protéine de fusion obtenue entre le fragment de la protéine 40 et l'élément immunogène peut également être fusionnée, par génie génétique à une protéine qui est un récepteur à une protéine sérique, en particulier à la sérumalbumine humaine.

L'élément immunogène, un antigène ou haptène, peut notamment provenir de virus ; on peut citer les protéines du RSV (Virus Respiratoire Syncitial) ou leurs fragments, par exemple la protéine G du RSV, ou l'antigène de l'hépatite B.

Dans le cas de la protéine G du RSV, on peut utiliser la protéine totale ou ses fragments, éventuellement modifiés par mutagénèse ponctuelle ou délétion.

La Demanderesse a montré que l'administration d'un haptène couplé à un fragment de la protéine P40 selon l'invention entrainait une augmentation susbtantielle de la réponse immunitaire, en limitant les risques de réactions à l'encontre de l'adjuvant lui-même.

Un procédé pour augmenter l'immunogénicité d'un antigène ou d'un haptène, caractérisé en ce qu'on associe ledit antigène ou haptène à un adjuvant qui comprend tout ou partie de la séquence de la protéine P40 de Klebsiella pneumoniae, sous forme d'un complexe tel que défini précédemment fait également partie de l'invention.

L'invention a donc également pour objet un vaccin, caractérisé en ce qu'il contient un élément immunogène associé à un fragment de la protéine P40 susceptible d'etre préparé par le procédé selon l'invention.

Elle comprend également des compositions pharmaceutiques contenant un complexe formé entre un adjuvant et un élément immunogène, tel que défini précédemment et des excipients pharmaceutiquement acceptables adaptés à son administration par voie parentérale et/ou orale.

Les exemples qui suivent sont destinés à illustrer l'invention sans aucunement en limiter la portée.

Dans ces exemples, on se référera aux figures suivantes :
- Figure 1 :: Stratégie de clonage par amplification génique de P40.
- Figure 2 :: Clonage de P40 dans pVABBG2ΔC.
- Figure 3 :: Choix des différents fragments de P40.
- Figure 4 :: Clonage de ΔP40G2ΔC dans pVABB
- Figure 5 :: Réponse anticorps anti-peptidique G1ΔC après des immunisations avec différentes concentrations de P40ext-G1ΔC.
- **Figure 6 :**: Réponse anticorps anti-peptidique G1ΔC obtenue avec différents schémas d'immunisation.

### Exemple 1 : Isolement et purification de la protéine p40

### Matériel et méthodes

La biomasse de Klebsiella pneumoniae (souche 1-145, 40 g de cellules sèches) est ajustée à pH 2,5 à l'aide d'acide acétique pur.

Après addition de 1/2 volume d'une solution contenant 6% cétrimide, 60% éthanol, 1,5 M CaCl₂ dont le pH est ajusté à 2,5 avec de l'acide acétique, le mélange est placé sous agitation pendant 16 heures à température ambiante.

Après centrifugation 20 mn à 15000 g à 4° C, les protéines du surnageant sont précipitées à l'éthanol. Deux précipitations successives avec centrifugation intermédiaire (10 mn, 10000 g, 4° C) sont réalisées : de 20 à 50 % puis de 50 à 80%.

Les culots obtenus après la seconde précipitation sont remis en suspension dans une solution de zwittergent 3-14, 1%.

Après agitation 4 heures à température ambiante, le pH est ajusté à 6,5 à l'aide de NaOH 1N.

Une centrifugation du mélange pendant 20 mn à 10000 g à 4° C permet d'obtenir une fraction enrichie en protéines membranaires (fraction MP).

Les protéines de la fraction MP sont dialysées contre un tampon Tris/HCl 20 mM pH 8,0 ; zwittergent 3-14, 0,1%. Le dialysat est déposé sur une colonne contenant un support de type échangeur d'anions forts (colonne de Ø = 50 mm x H = 250 mm, gel Biorad Macroprep High Q) équilibrée dans le tampon décrit ci-dessus. La protéine P40 est éluée pour une concentration de 50 mM en NaCl dans le tampon d'équilibration.

Les fractions contenant la P40 sont rassemblées et dialysées contre un tampon citrate 20 mM pH 3,0 ; zwittergent 3-14, 0,1%. Le dialysat est déposé sur une colonne contenant un support de type échangeur de cations forts (dimensions de la colonne : Ø = 25 mm x H = 160 mm, gel Biorad Macroprep High S) équilibrée dans le tampon citrate 20 mM pH 3,0, zwittergent 3-14, 0,1 %. La protéine P40 est éluée pour une concentration 0,7 M en NaCl. Les fractions contenant la P40 sont rassemblées et concentrées par ultrafiltration à l'aide d'un système de filtration à flux tangentiel Minitan Millipore utilisé avec des plaques de membranes possédant un seuil de coupure 10 kDa.

### Résultats

Les fractions obtenues après chaque étape chromatographique sont analysées par SDS-PAGE afin de rassembler celles contenant la protéine P40.

Les quantités de protéines sont mesurées par la méthode de Lowry (tableau 1).

**Tableau 1 :**

| **Tableau récapitulatif des quantités de protéine et LPS des fractions obtenues pour les différentes étapes du procédé de purification de la protéine P40 (n.d. = non déterminé)** | | | |
|---|---|---|---|
| | Protéines | Rendement | LPS |
| Biomasse | 40 g | - | n.d. |
| Fraction MP | 900 mg | 2,25 % | n.d. |
| Fraction enrichie en P40 | 400 mg | 1 % | 10 % |
| Protéine P40 | 130 mg | 0,3 % | < 1% |

La pureté et l'homogénéité de la protéine P40 sont estimées par SDS-PAGE.

Après l'étape de chromatographie d'échange de cations, la protéine P40 est dépourvue du contaminant majeur présent dans la fraction MP (la protéine présentant une masse moléculaire apparente de 18 kDa) et présente un degré de pureté supérieur à 95%. D'autre part, cette étape de purification permet l'élimination des lipopolysaccharides. Cette étape de purification n'existait pas dans le procédé de purification précédemment présenté.

Le profil électrophorétique de la P40 révèle plusieurs bandes. Ces bandes sont reconnues après immunoblot par des anticorps monoclonaux anti-P40 obtenus chez la souris. La bande majeure supérieure correspond à la protéine dénaturée (par le traitement à 100 °C, 15 min. en présence de SDS), et la bande mineure inférieure à la protéine sous sa forme native.

La P40 est en effet une protéine dite modifiable par la chaleur (heat-modifiable), et cette propriété à été vérifiée à l'aide d'une cinétique de chauffage à 100°C en présence de SDS. Sans chauffage la protéine sous forme native présente une structure en feuillets β-qui fixe plus de SDS et migre donc plus loin vers l'anode que la forme dénaturée (dénaturation complète après 5 min. à 100 ° C) qui présente une structure en hélices a (KELLER, K. B. *1978*, J. Bacteriol., 134, 1181-1183).

La contamination par les lipopolysaccharides (LPS) est estimée par dosage par chromatographie en phase gazeuse de l'acide β-hydroxymyristique, acide gras marqueur des LPS de Klebsiella pneumoniae (tableau 1).

Cette méthode ne peut être utilisée que pour approcher la teneur en LPS des échantillons issus des différentes étapes de purification.

La quantité d'acide β -hydroxymyristique présente dans la fraction P40 après chromatographie d'échange de cations étant inférieure au seuil de quantification du dosage, on peut estimer que la quantité de LPS résiduel est inférieure à 1%.

### Exemple 2 : Clonage et expression de la protéine P40

72% de la séquence du gène de l'OmpA de Klebsiella pneumoniae a été publié par LAWRENCE et al, 1991, J. Gen. Mlcrobiol., 137 : 1911-1921).

L'originalité de nos travaux réside dans la détermination de la totalité de la séquence, soit celle correspondant aux 83 acides aminés N-terminaux et aux 11 acides aminés C-terminaux (sur un total de 335 acides aminés).

### Matériel et méthode

### Souches bactériennes

* E. coli : RV 308 : souche ATCC 31608 (Maurer, R. et al., *1980,* J. Mol. Biol., 139, 147-161).
* K. pneumoniae : IP 145 : souche C.I.B.P.F - Brevet d'invention déposé le 19 janvier 1981.

### Vecteurs

* pRIT 28 (Hultman T. et al., *1988* , Nucléosides Nucléotides, 7 : 629-638) : vecteur de clonage et de séquençage possédant le gène de résistance à l'ampicilline, les origines de réplication d'E coli et du phage F1 ainsi qu'une portion du gène lac-Z d'E. coli (β-galactosidase).
* pVABB : vecteur d'expression de fusion de gène.

### Solutions

* **Amplification génique**

| | |
|---|---|
| Tampon de lyse | 25 mM Taps pH 9,3 |
| | 2 mM MgCl₂ |
| Tampon d'amplification | 25 mM Taps pH 9,3 |
| | 2 mM MgCl₂ |
| | Tween 20 0,1 % |
| | 200 mM dNTP. |

* **Purification des protéines**

| | | | |
|---|---|---|---|
| TST (20X) | Tris base | 0,5 M | |
| | HCl | 0,3 M | |
| | NaCl | 4 M | |
| | Tween 20 | 1% | |
| | EDTA | 20 mM | |
| | | | |
| Tampon de lavage | Tris HCl | 50 mM | pH 8,5 |
| | MgCl₂ | 5 mM | |
| | | | |
| Solution de dénaturation | Gua-HCl | 7,8 M | |
| | Tris-HCl | 28 mM | pH 8,5 |
| | | | |
| Solution de renaturation | Gua-HCl | 0,5 M | |
| | Tris-HCl | 25 mM | pH 8,5 |
| | NaCl | 150 mM | |
| | Tween 20 | 0,05 %. | |

### Synthèse des oligonucléotides

Les amorces nucléotidiques ont été déterminées à partir de la partie de la séquence publiée de l'OMPA de Klebsiella pneumoniae (Lawrence, J.G. et al., *1991*, J. Gen. Microbiol., 137: 1911-1921) de la séquence conscensus issue de l'alignement des séquences de 5 OMPA d'entérobactéries (E. coli, S. typhimurium, S. marcescens, S. dysenteriae, E. aeroginosae), ainsi que des séquences de peptides obtenus par séquençage manuel.

Les oligonucléotides ont été synthétisés selon la méthode chimique des phosphoramidites sur l'appareil "Gene Assembler Plus" de Pharmacia.

### Amplification génique par PCR du gène P40

L'ADN de l'OMPA de Klebsiella pneumoniae a été amplifié de la manière suivante.

Une colonie de Klebsiella pneumoniae est lysée dans 10 µl de tampon de lyse par chauffage à 95° C pendant 5 minutes.

1 µl de cette solution sert de source d'ADN pour les réactions d'amplification.

Celles-ci sont réalisées dans 100 µl de tampon d'amplification, avec 5 pmoles de chaque amorce et une unité d'enzyme Taq polymérase (Perkin Elmer Cetus). Chaque cycle comprend une étape de dénaturation de 30 secondes à 95° C suivie d'une hybridation de l'amorce à l'ADN et d'une extension d'une minute à 72° C. 30 cycles sont ainsi effectués à l'aide d'un thermocycleur "Gen Amp PCR" 9000 Perkin Elmer Cetus.

Les PCR suivantes sont réalisées à partir des fragments d'ADN amplifiés précédemment.

Les fragments d'ADN amplifiés sont ensuite digérés et liés au vecteur PRIT 28.

### Séquençage

Les fragments ainsi clonés sont séquencés sur un séquenceur automatique 373 DNA Séquenceur d'Applied Biosystem. Les réactions de séquençage sont réalisées à l'aide du kit "dye Terminator" selon les recommandations du fournisseur (Applied Biosystem) soit sur de l'ADN double brin obtenu après amplification génique ou issu de maxiprep soit sur de l'ADN simple brin issu de fragments PCR dénaturés (Hultman, T. et al, *1989*, Nucleid Acids Rev. 17 : 4937-4946).

### Expression de la protéine

Le gène entier de P40 est cloné dans le vecteur d'expression pVABB. Ce vecteur permet d'adjoindre une queue d'affinité "BB" à P40 ; B étant la partie de la protéine G du streptocoque qui lie la serumalbumine (Nygren, P.A. et al, *1988*, J. Mol. Recognit. 1, 69-74).

Les souches d'E. coli RV308 transformées par le vecteur pVABBP40 sont mises à cultiver une nuit à 37° C sous agitation, dans 100 ml de TSB complémenté en extrait de levure, en ampicilline (200 µg/ml) en tétracycline (8 µg/ml) et en tryptophane (100 µg/ml). Le lendemain, une culture à D0 = 1 pour une longueur d'onde de 580 nm est préparée dans du TSB + extraits de levure + ampi + tetra.

Après 10 minutes de culture, l'expression de la protéine est induite par addition d'IAA à (25 µg/ml) dans le milieu. La culture est centrifugée à 4° C à 2460 g pendant 10 minutes.

Le culot est repris par 20 ml de TST 1 x pH 7,4, et la solution est alors centrifugée à 4° C à 23000 g pendant 30 minutes.

Le surnageant est passé sur HSA Sépharose ce qui permet d'isoler les protéines dites solubles. Le culot est lavé avec du tampon de lavage puis centrifugé à 23000 g à 4° C pendant 30 minutes. Le culot renfermant les corps d'inclusion est alors repris par 900 µl d'une solution dénaturante + 100 µl de Dithiothreitol 10 mM et incubé 2 heures à 37° C.

La solution est ensuite incubée 1 nuit à température ambiante, sous agitation, dans 100 ml de tampon de renaturation puis centrifugée à 23 000 g à 4°C pendant 30 minutes.

Le surnageant est passé sur HSA Sépharose.

Dans les deux cas les protéines fixées sont éluées avec de l'acide acétique 0,5 M pH 2,8 et collectées par fraction de 1 ml.

Les fractions collectées sont ensuite analysées sur gel d'électrophorèse en SDS-PAGE et par Immuno blot.

### Résultats

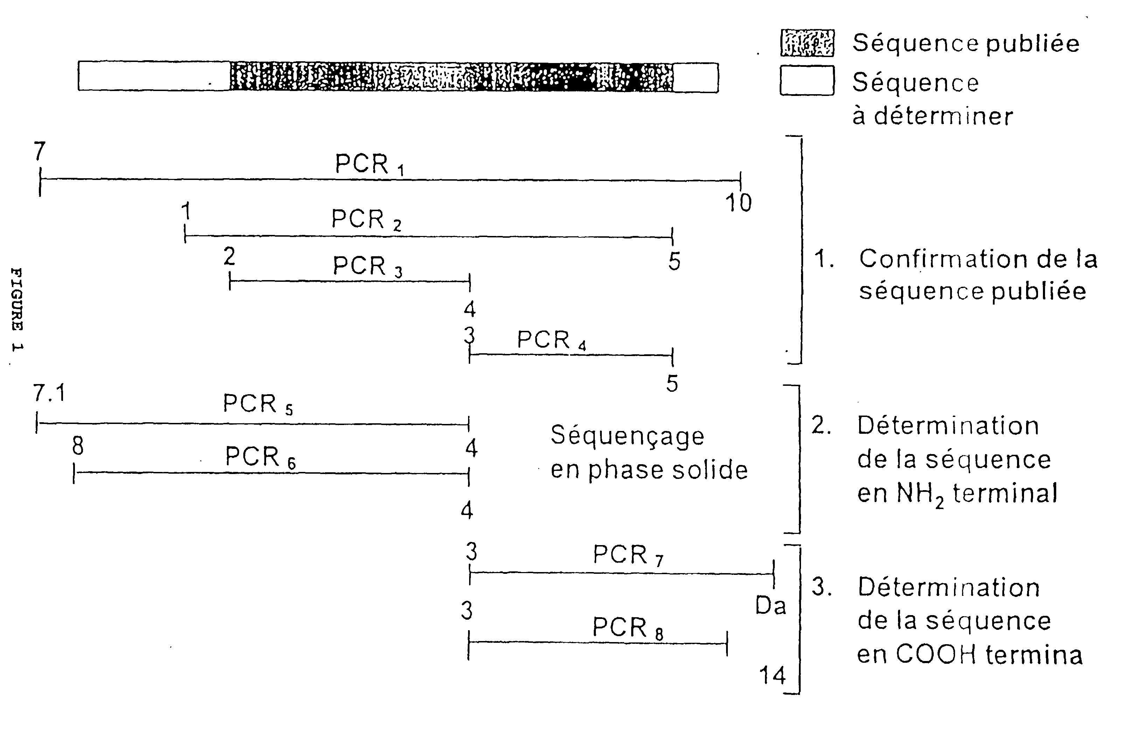

Le clonage du gène a été effectué en trois temps selon la stratégie présentée sur la figure 1.

Dans un premier temps, nous avons confirmé la partie de la séquence publiée à l'exception d'un T à la place d'un A en position 103.

Puis nous avons déterminé la séquence en 3' du gène et enfin celle en 5'.

Le gène entier a été obtenu par fusion des deux parties 8/4 et 3/14 puis cloné dans le vecteur pRIT 28. La séquence est la séquence id n° 1.

La protéine est exprimée sous la forme BBP40.

Elle est essentiellement obtenue à partir des corps d'inclusion. Pour une culture de 200 ml, on purifie une quinzaine de milligrammes de protéine.

Le profil électrophorétique montre que BBP40, obtenue après dénaturation, est d'une grande pureté. Le poids moléculaire apparent, correspond au poids théorique calculé qui est de 63 kDa.

La caractérisation en Immuno blot montre que la protéine purifiée est bien reconnue par un sérum de lapin anti-P40.

### Exemple 3 : Protéine de fusion BBP40G2ΔC, sous groupe a

Un oligonucléotide correspondant à la partie N Terminale délétée du codon stop du gène, a été synthétisé.

La partie en 5' a été amplifiée par PCR, purifiée, clonée dans le vecteur pRIT 28 et séquencée, selon la méthodologie décrite dans l'exemple 2.

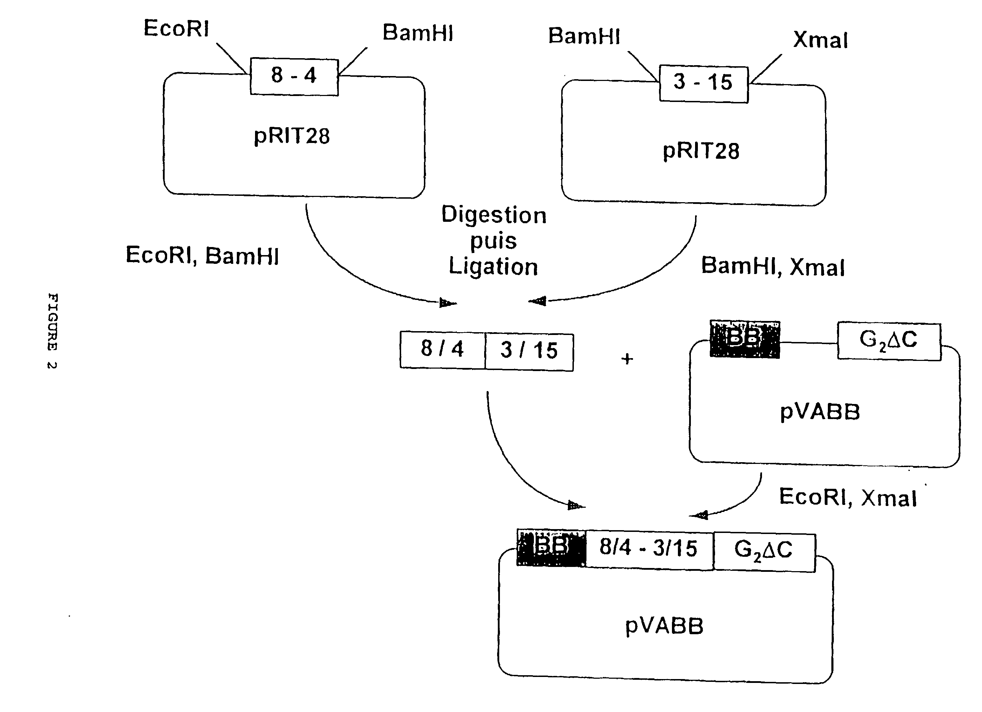

Dans un deuxième temps, les deux parties du gène ont été fusionnées et clonées dans le vecteur pVABBG2ΔC (figure n° 2). G2ΔC représente la séquence d'un fragment de 101 amino-acides de la protéine G du virus respiratoire syncytial G (130-230).

Des bactéries E. coli de la souche RV308 sont ensuite transformées avec le vecteur PVABBG2ΔC.

Les protéines produites sont purifiées comme déjà décrit pour BBP40.

### Résultats

La protéine BBP40G2ΔC est essentiellement obtenue à partir des corps d'inclusion. On purifie une douzaine de mg de protéines à partir de 200 ml de milieu de culture.

En électrophorèse, la protéine est assez pure.

La masse moléculaire apparente correspond à la masse théorique calculée qui est de 75 kDa.

### Exemple 4 : Clonage et expression de trois fragments de P40

### Matériel et méthodes

### Les oligonucléotides

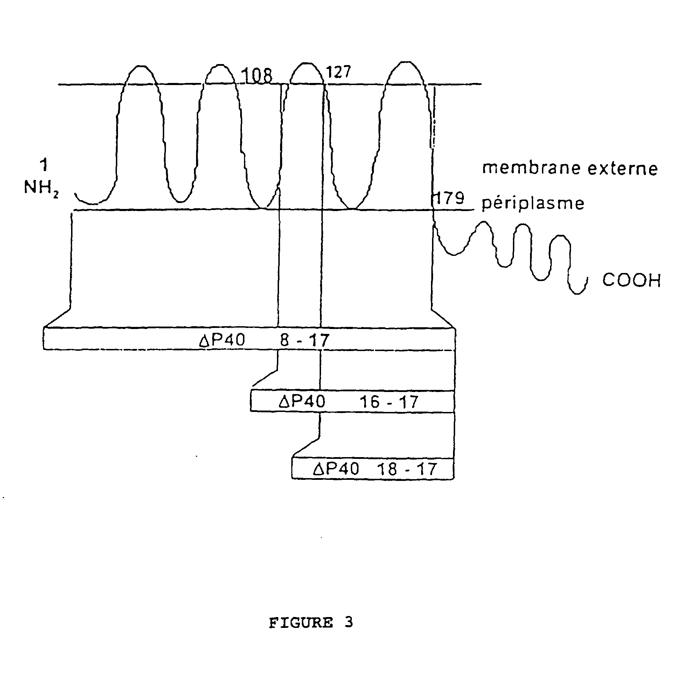

Trois oligonucléotides complémentaires de la séquence de P40 ont été synthétisés : 16-17-18 (cf. figure 3).

Des parties du gène déterminées ont ensuite été amplifiées en PCR à partir de l'ADN d'une miniprep (protocole Applied) de pRIT 28 P40.

On a ainsi pu cloner la partie du gène correspondant à la totalité de la partie transmembranaire (8/17, baptisé fragment n° 8) à deux boucles externes-deux portions transmembranaires (16/17, baptisé fragment n°16) et 1 boucle externe deux portions transmembranaires (18/17, baptisé fragment n° 18).

Les fragments d'ADN ainsi amplifiés sont digérés puis isolés et ligués au vecteur pRIT 28 et séquencés (cf. BBP40 clonage de P40).

### La protéine de fusion BBΔP40G2ΔC

Le gène G2ΔC est digéré à partir du vecteur pRIT 28 G2ΔC puis ligué au vecteur digéré pRIT 28 ΔP40 (ΔP40 représente un des fragments de P40).

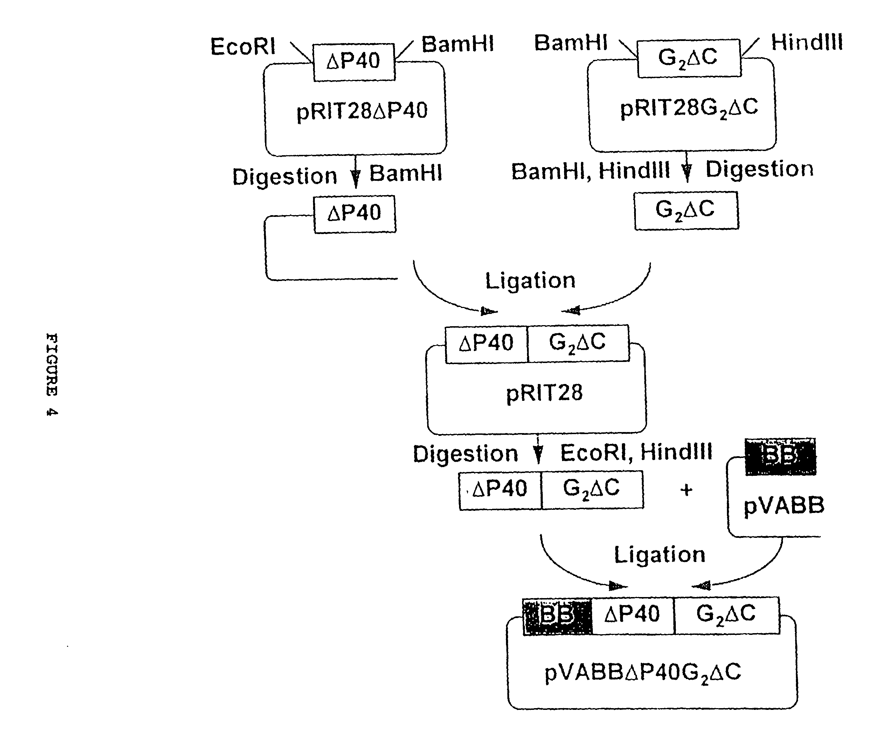

Ensuite, l'ensemble ΔP40G2ΔC est digéré et cloné dans pVABB (cf. figure 4).

Les trois protéines hybrides sont exprimées selon le protocole décrit pour BBP40.

### Résultats

Tout comme BBP40 et BBP40G2ΔC, BB8G2ΔC est obtenu essentiellement à partir des corps d'inclusion. Une culture de 400 ml donne une dizaine de mg de protéines.

Par contre, les protéines BB18G2ΔC, et BB16G2ΔC se retrouvent majoritairement à l'étape de sonication, sous forme soluble. Dans les deux cas, on obtient une dizaine de mg/400 ml de culture.

Ces protéines ont été caractérisées en électrophorèse SDS-PAGE. Leur masse moléculaire correspond à la masse théorique calculée :

| | |
|---|---|
| BB8G2ΔC | 58,03 kDa |
| BB16G2ΔC | 46,5 kDa |
| BB18G2ΔC | 45,5 kDa |

Les trois hybrides sont reconnus aussi bien par un anticorps polyclonal anti- G2 qu'anti P40 en Western Blot.

### Exemple 5

### 1. Effets de la protéine P40 sur des cellules du système immunitaire

### 1.a. Lymphocytes B

On a injecté par voie sous-cutanée à des souris BALB/c (5 par groupe) aux jours 0 et 21, 30 µg de P40 obtenus par extraction de la membrane (P40 ext) ou par recombinaison génétique (P40 rec, c'est-à-dire BBP40). Les immunisations ont été effectuées sans aucun adjuvant. 10 jours après la dernière immunisation, la réponse en anticorps anti-P40ext a été évaluée sur les sérums individuels par la méthode ELISA. Le tableau 2 donne la moyenne des titres obtenus sur 5 échantillons. Les contrôles négatifs ne contenaient pas d'anticorps anti-P40ext.

**Tableau 2 :**

| **réponse anticorps anti-P40ext** | | |
|---|---|---|
| Immunisations avec: | xtP40 | recP40 |
| Titres d'anticorps : | 87040 | 112640 |

Dans ces conditions expérimentales, la P40rec est aussi immunogène que la P40ext. Ces deux protéines contiennent donc des épitopes B qui interagissent avec les lymphocytes B.

### 1.b. Lymphocytes T

La réaction d'hypersensibilité retardée (HSR) à la P40ext a été mesurée par le test du gonflement différé du coussinet. Des souris BALB/c (5 par groupe) ont été sensibilisées par voie sous-cutanée avec 100 µg de P40ext sans le moindre adjuvant. Après 6 à 10 jours, les souris ont été stimulées par voie sous-cutanée avec 100 µg de P40ext/20 µl dans le coussinet postérieur droit, alors que le coussinet postérieur gauche recevait du PBS. 24 heures plus tard, le gonflement du coussinet a été mesuré. On n'observe pas d'hypersensibilité retardée dans le contrôle négatif (5 souris non sensibilisées).

**Tableau 3 :**

| **réaction d'hypersensibilité retardée induite par P40ext, mesurée par le gonflement du coussinet (en mm)** | | | |
|---|---|---|---|
| J6 | | J10 | |
| BALB/c | C57B1/6 | BALB/c | C57Bl/6 |
| 7,9 | 7,8 | 7,5 | 7,4 |

Les résultats montrés dans le tableau 3 indiquent que les souris immunisées avec P40ext produisent des réactions d'hypersensibilité retardée hautement quantitatives dans le coussinet. La réaction HSR reflète la réponse immunitaire à médiation cellulaire, nécessitant des cellules Th1. On peut en conclure que P40 ext contient au moins un épitope T qui est capable de favoriser la réponse Th1, sans restriction MHC.

### 1.3. Macrophages

L'effet de P40ext sur des macrophages a été déterminé par leur production de nitrite. Des cellules RAW 264,7, qui sont des monocytes-macrophages de souris, ont été incubées 72 heures à 37° C en présence de différentes concentrations de P40ext. La quantité de nitrites dans le surnageant des cultures cellulaires a été mesurée par un dosage colorimétrique avec le réactif de Griess-Ilosvay.

La production de nitrite reflète l'activation des macrophages, et joue un rôle crucial dans l'activité anti-microbienne et anti-tumorale de ces cellules. Les données obtenues montrent que P40ext stimule la production de nitrite des cellules RAW 264,7, démontrant que P40ext active les macrophages.

### 2. P40 est un porteur à effet adjuvant pour un peptide (G1ΔC)

### 2.1. Comparaison de P40ext avec d'autres supports

Le peptide utilisé est G1ΔC, un peptide obtenu à partir de la protéine G du RSV : (G174-187 ΔC) Trudel et al., *1991*, J. Virol. 185: 749-757.

### Cinétique de la réponse immunitaire contre G1 ΔC

Des souris C57B1/6 (5 par groupe) sont immunisées avec G1 ΔC sous différentes formes selon un schéma d'immunisation identique. Les réponses anticorps induites par les différentes formes de G1 ΔC sont comparées dans le temps : 7, 17, 28, 35, 42 jours après le début de l'expérience.

La réponse anti-G1 ΔC est significativement plus élevée et plus rapide lorsque les souris sont immunisées avec P40/G1 ΔC que les immunisations plus classiques TT/G1 ΔC et KLH/G1 ΔC+AF. Une seule injection de P40/G1 ΔC permet d'obtenir, en 7 jours, un titre d'anticorps anti-G1 ΔC de 1000. Ce titre est obtenu avec TT/G1 ΔC+AF en 28 jours. La réponse maximum (titre = 1/380000), obtenue après trois injections, en 28 jours, est environ 30 fois supérieure à celle obtenue avec KLH/G1 ΔC +AF et 70 fois supérieure à celle obtenue avec TT/G1 ΔC. Le titre en anticorps anti-G1 se maintient sans faiblir jusqu'au jour 42.

### Conclusion

Le couplage chimique du peptide G1 ΔC sur la protéine P40 a permis d'induire une réponse anti-G1 ΔC aussi importante que les modèles de référence KLH/G1 ΔC+AF ou TT/G1 ΔC.

Les résultats obtenus montrent que P40ext est une molécule porteuse à effet adjuvant pour G1ΔC : P40ext est meilleure que la toxine tétanique et aussi bonne que l'association KLH + adjuvant de Freund.

### 2.1. Distribution isotypique des anticorps anti-peptide G1ΔC

Les isotypes des sérums obtenus pendant les expériences décrites ci-dessus ont été déterminés par ELISA. Le tableau 4 présente la moyenne des valeurs de A450 de 5 sérums individuels testés à la dilution 1/250.

**Tableau 4 :**

| **distribution isotypique des anticorps anti-peptide G1ΔC** | | | | |
|---|---|---|---|---|
| | IgG1 | IgG2a | IgG2b | IgG3 |
| A450 (dil.1/250) | 2,892 | 1,212 | 2,970 | 0,209 |

On a montré que la sécrétion d'isotype d'anticorps est régulée par des sous-ensembles de cellules Th spécifiques d'un antigène qui peuvent être divisés en deux sous- ensembles Th1 et Th2. Les clones Th1 produisent de l'Il-2 et IFN-gamma, et des lymphotoxines, alors que les clones Th2 produisent de l'Il-4 et de l'Il-5. Les clones de Th1 et de Th2 induisent specifiquement la sécrétion par les cellules B spécifiques d'un antigène, respectivement d'IgG2a + IgG3 et d'IgG1 + IgG2b + IgE. Les données présentées dans le tableau 4 montrent que IgG1 et IgG2b sont les deux isotypes majeurs des anticorps anti G1ΔC, l'IgG2a étant également représenté. On peut en conclure que chez les souris C57B1/6, P40-G1ΔC provoque une réponse Th2 supérieure à la réponse Th1.

### 2.2. Etude dose-effet

On a injecté par voie sous-cutanée à des souris BALB/c (5 par groupe) différentes concentrations de P40ext-G1ΔC, aux jours 0, 10 et 21. Une semaine après la dernière immunisation, des échantillons de sang sont prélevés et la réponse anticorps anti-peptide G1ΔC est estimée sur les sérums individuels par ELISA. La moyenne des titres de 5 échantillons est effectuée.

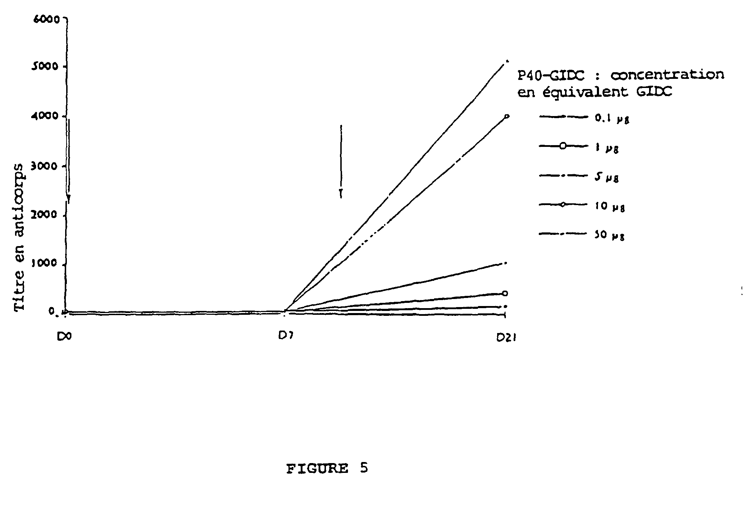

La figure 5 montre le rapport dose-effet de P40ext-G1ΔC. Une réponse anticorps anti-peptide G1ΔC est obtenue avec 1 µg de P40ext-G1ΔC. Les titres en anticorps les plus élevés sont observés avec 10 à 50 µg de P40ext-G1ΔC.

### 2.4. Détermination du schéma d'immunisation optimale

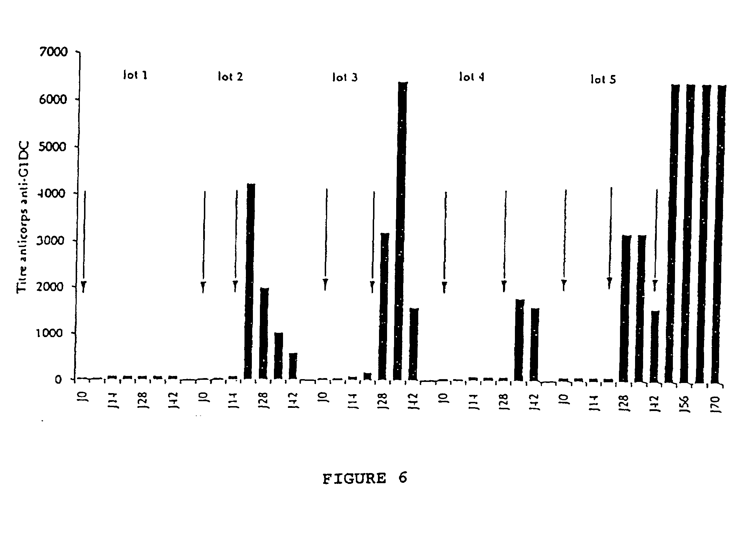

On a injecté par voie sous-cutanée à des souris BALB/c (5 par groupe) P40ext-G1ΔC (équivalent à 10 µg de G1 ΔC) aux jours indiqués sur la figure 6. La réponse anticorps anti-peptide G1ΔC est déterminée sur les sérums individuels par ELISA. 4 schémas d'immunisations ont été testés : une injection, deux injections aux jours 0 et 14, ou aux jours 0 et 21, et trois injections aux jours 0, 21 et 40. La réponse anticorps anti-peptide anti-G1ΔC la plus élevée est obtenue avec trois injections.

### 3. P40ext est un adjuvant efficace pour un antigène protéique (BBG2ΔC)

On a injecté par voie sous-cutanée à des souris BALB/c (5 par groupe) BBG2ΔC conjugué chimiquement à P40ext (équivalent à 10 µg de G2 ΔC) aux jours 0 et 21. Dix jours plus tard, la réponse anticorps anti-G2ΔC est déterminée dans les sérums individuels par ELISA. Les moyennes des titres de 5 échantillons sont données dans le tableau 5. Le contrôle négatif ne contenait pas d'anticorps anti-G2ΔC.

**Tableau 5 :**

| **effet adjuvant de P40ext sur un antigène protéique** | |
|---|---|
| | Titre en anticorps anti-G2ΔC |
| BBG2ΔC | 160 |
| BBG2ΔC + adjuvant de Freund | 2051200 |
| extP40-BBG2ΔC | 29800 |

BBG2ΔC est faiblement immunogène. L'utilisation d'adjuvant de Freund augmente le titre en anticorps anti-G2ΔC. Quand BBG2ΔC est conjugué par voie chimique à P40ext, la réponse anticorps anti-G2ΔC est augmentée d'environ 200 fois. P40ext est donc un bon adjuvant pour un antigène protéique.

### 4. Activité adjuvante des fragments de P40

On a injecté par voie sous-cutanée à des souris BALB/c (5 par groupe) au jour 0 et stimulé au jour 21 par les protéines recombinantes suivantes : la protéine de fusion BBP40G2ΔC, la protéine de fusion contenant le fragment de P40 n° 8 (BB8G2ΔC), la protéine de fusion contenant le fragment de P40 n° 16 (BB16G2ΔC) et la protéine de fusion contenant le fragment de P40 n° 18 (BB18G2ΔC) ( 10 µg équivalent G2 ΔC).

Au jour 31, les réponses anticorps anti-G2ΔC, anti-P40 et anti-BB sont déterminées par ELISA dans les sérums individuels. La moyenne des titres de 5 sérums individuels est effectuée. Les contrôles négatifs ne contenaient pas d'anticorps anti-G2ΔC.

**Tableau 6 :**

| effet adjuvant des fragments recombinants de P40 . | | | |
|---|---|---|---|
| | TITRE EN ANTICORPS ANTI-G2ΔC | TITRE EN ANTICORPS ANTI-BB | TITRE EN ANTICORPS ANTI-P40 |
| BBP40G2ΔC | 14 800 | 266 240 | 450 506 |
| BB8G2ΔC | 7 400 | 430080 | 56 640 |
| BB16G2ΔC | 1 800 | 84480 | 880 |
| BB18G2ΔC | 1 360 | 184 320 | 240 |

Dans cette expérience, on montre que les fragments de P40 conservent les propriétés de la protéine entière. Ceci est particulièrement spectaculaire lorsqu'on examine la réponse anticorps anti-BB.

La réponse anticorps anti-P40 est considérablement réduite en utilisant les fragments de P40.

## Revendications

1. Produit adjuvant destiné à améliorer l'activité d'une molécule lors de l'administration à un hôte, **caractérisé en ce qu'**il comprend au moins la partie consistant en la séquence comprise entre les amino-acides 1 à 179, 108 à 179 ou 127 à 179 de la protéine P40 de Klebsiella pneumoniae de séquence SEQ ID N°2.

2. produit adjuvant selon la revendication 1, **caractérisé en ce qu'**il comprend une protéine présentant la séquence ID n° 2 ou présentant au moins 90% d'homologie avec cette séquence.

3. Produit adjuvant selon l'une des revendications 1 ou 2, **caractérisé en ce qu'**il consiste en la séquence comprise entre les amino-acides 1 à 179 de la protéine P40 de K. pneumoniae, ou une séquence présentant au moins 90% d'homologie avec la séquence comprise entre les amino-acides numérotés 1 et 179 de la séquence de la protéine P40 de K. pneumoniae.

4. Produit adjuvant selon l'une des revendications 1 ou 2, **caractérisé en ce qu'**il consiste en la séquence comprise entre les amino-acides 108 à 179 de la protéine P40 de K.pneumoniae ou une séquence présentant au moins 80% d'homologie avec la séquence comprise entre les amino-acides numérotés 108 et 179 de la protéine P40 de K. pneumoniae.

5. Produit adjuvant selon l'une des revendications 1 ou 2, **caractérisé en ce qu'**il consiste en la séquence comprise entre les amino-acides numérotés 127 à 179 de la protéine P40 de K. pneumoniae ou une séquence présentant au moins 80% d'homologie avec la séquence comprise entre les amino-acides numérotés 127 à 179 de la peotéine P40 de K. pneumoniae.

6. Protéine ou peptide présentant l'une des séquences ID n° 2, ID n° 4, ID n° 6 ou ID n° 8.

7. Complexe immunogène du type comprenant un élément immunogène, associé à un adjuvant augmentant l'intensité de la réponse immunitaire, **caractérisé en ce que** l'élément immunogène est un antigène ou un haptène, et l'adjuvant comprend un produit selon l'une des revendications 1 à 6.

8. Complexe immunogène selon la revendication 7, **caractérisé en ce que** l'élément immunogène est associé à l'adjuvant par une liaison covalente.

9. Complexe immunogène selon l'une des revendications 7 ou 8, **caractérisé en ce que** l'élément immunogène est constitué d'un fragment de la protéine G du RSV.

10. Complexe immunogène selon l'une des revendications 7 à 9, **caractérisé en ce que** l'élément immunogène, associé à l'adjuvant, est fusionné avec une protéine qui est un récepteur à une protéine sérique, en particulier à la sérumalbumine humaine.

11. Procédé in vitro pour augmenter l'immunogénicité d'un antigène ou d'un haptène, **caractérisé en ce qu'**on associe ledit antigène ou haptène à un adjuvant selon l'une des revendications 1 à 6, sous forme d'un complexe selon l'une des revendications 7 à 10.

12. Procédé selon la revendication 11, **caractérisé en ce qu'**on associe l'antigène ou l'haptène à l'adjuvant par couplage chimique.

13. Procédé selon l'une des revendications 11 ou 12, **caractérisé en ce que** l'antigène ou l'haptène est fusionné par génie génétique à l'adjuvant.

14. Vaccin **caractérisé en ce qu'**il contient un complexe selon l'une des revendications 7 à 10, susceptible d'être préparé par le procédé selon l'une des revendications 11 à 13.

15. Procédé de préparation d'un produit adjuvant, protéine ou peptide selon l'une des revendications 1 à 6, à partir de membranes de bactéries du genre Klebsiella pneumoniae, **caractérisé en ce qu'**il comprend les étapes de :
a) précipitation des lipopolysaccharides par addition de détergent et d'un sel de cation divalent et récupération du surnageant,
b) précipitation des protéines du surnageant et remise en suspension du culot.
c) chromatographie de la suspension sur échangeur d'anions et récupération des fractions contenant le produit adjuvant,
d) chromatographie sur échangeur de cations et récupération de la fraction contenant le produit adjuvant.
e) concentration de la fraction obtenue à l'issue de l'étape d) pour récupérer un produit adjuvant sous forme de protéine, essentiellement dépourvu de liposaccharides.

## Claims

1. Adjuvant product intended to improve the activity of a molecule when administered to a host, **characterized in that** it comprises at least the part consisting of the sequence encompassed between amino acids 1 to 179, 108 to 179 or 127 to 179 of the P40 protein of Klebsiella pneumoniae having the sequence ID No. 2.

2. Adjuvant product according to Claim 1, **characterized in that** it comprises a protein having the sequence ID No. 2 or having at least 90% homology with this sequence.

3. Adjuvant product according to Claim 1 or 2, **characterized in that** it consists of the sequence encompassed between amino acids 1 to 179 of the P40 protein of K. pneumoniae, or a sequence having at least 90% homology with the sequence encompassed between amino acids nos. 1 and 179 of the sequence of the P40 protein of K. pneumoniae.

4. Adjuvant product according to Claim 1 or 2, **characterized in that** it consists of the sequence encompassed between amino acids 108 to 179 of the P40 protein of K. pneumoniae or a sequence having at least 80% homology with the sequence encompassed between amino acids nos. 108 and 179 of the P40 protein of K. pneumoniae.

5. Adjuvant product according to Claim 1 or 2, **characterized in that** it consists of the sequence encompassed between amino acids nos. 127 to 179 of the P40 protein of K. pneumoniae or a sequence having at least 80% homology with the sequence encompassed between amino acids nos. 127 to 179 of the P40 protein of K. pneumoniae.

6. Protein or peptide having one of the sequences ID No. 2, ID No. 4, ID No. 6 or ID No. 8.

7. Immunogenic complex of the type which comprises an immunogenic element which is attached to an adjuvant which increases the strength of the immune response, **characterized in that** the immunogenic element is an antigen or a hapten, and the adjuvant comprises a product according to one of Claims 1 to 6.

8. Immunogenic complex according to Claim 7, **characterized in that** the immunogenic element is attached to the adjuvant by a covalent bond.

9. Immunogenic complex according to Claim 7 or 8, **characterized in that** the immunogenic element consists of a fragment of the G protein of RSV.

10. Immunogenic complex according to one of Claims 7 to 9, **characterized in that** the immunogenic element, which is attached to the adjuvant, is fused to a protein which is a receptor for a serum protein, in particular for human serum albumin.

11. In vitro process for increasing the immunogenicity of an antigen or a hapten, **characterized in that** the said antigen or hapten is attached to an adjuvant according to one of Claims 1 to 6 in the form of a complex according to one of Claims 7 to 10.

12. Process according to Claim 11, **characterized in that** the antigen or hapten is attached to the adjuvant by chemical coupling.

13. Process according to Claim 11 or 12, **characterized in that** the antigen or hapten is fused to the adjuvant by genetic manipulation.

14. Vaccine, **characterized in that** it contains a complex according to one of Claims 7 to 10 which may be prepared by the process according to one of Claims 11 to 13.

15. Process for preparing a protein or peptide adjuvant product according to one of Claims 1 to 6 from membranes of bacteria of the genus Klebsiella pneumoniae, **characterized in that** it comprises the steps of :
a) precipitating the lipopolysaccharides by adding detergent and a salt of a divalent cation and recovering the supernatant,
b) precipitating the proteins from the supernatant and resuspending the pellet,
c) chromatographing the suspension on an anion exchanger and recovering the fractions which contain the adjuvant product,
d) chromatographing on a cation exchanger and recovering the fraction which contains the adjuvant product,
e) concentrating the fraction obtained from step d) in order to recover an adjuvant product in the form of protein which is essentially free of liposaccharides.

## Patentansprüche

1. Adjuvans-Erzeugnis, welches dazu bestimmt ist, die Aktivität eines Moleküls während der Verabreichung an einen Wirt zu erhöhen, **dadurch gekennzeichnet, dass** es wenigstens den aus der Sequenz zwischen den Aminosäuren 1 bis 179, 108 bis 179 oder 127 bis 179 eingeschlossen bestehenden Teil des Proteins P40 von Klebsiella pneumoniae mit der Sequenz SEQ ID Nr. 2 umfasst.

2. Adjuvans-Erzeugnis nach Anspruch 1, **dadurch gekennzeichnet, dass** es ein Protein umfasst, welches die Sequenz ID Nr. 2 aufweist oder wenigstens 90% Homologie zu dieser Sequenz aufweist.

3. Adjuvans-Erzeugnis nach einem der Ansprüche 1 oder 2, **dadurch gekennzeichnet**, das es aus der Sequenz zwischen den Aminosäuren 1 bis 179 eingeschlossen des Proteins P40 von K. pneumoniae oder einer Sequenz, welche wenigstens 90% Homologie zu der Sequenz zwischen den Aminosäuren mit den Nummern 1 und 179 eingeschlossen der Sequenz des Proteins P40 von K. pneumoniae aufweist, besteht.

4. Adjuvans-Erzeugnis nach einem der Ansprüche 1 oder 2, **dadurch gekennzeichnet, dass** es aus der Sequenz zwischen den Aminosäuren 108 bis 179 eingeschlossen des Proteins P40 von K. pneumoniae oder einer Sequenz, welche wenigstens 80% Homologie zu der Sequenz zwischen den Aminosäuren mit den Nummern 108 und 179 eingeschlossen des Proteins P40 von K. pneumoniae aufweist, besteht.

5. Adjuvans-Erzeugnis nach einem der Ansprüche 1 oder 2, **dadurch gekennzeichnet, dass** es aus der Sequenz zwischen den Aminosäuren mit den Nummern 127 bis 179 eingeschlossen des Proteins P40 von K. pneumoniae oder einer Sequenz, welche wenigstens 80% Homologie zu der Sequenz zwischen den Aminosäuren mit den Nummern 127 bis 179 eingeschlossen des Proteins P40 von K. pneumoniae aufweist, besteht.

6. Protein oder Peptid, welches eine der Sequenzen ID Nr. 2, ID Nr. 4, ID Nr. 6 oder ID Nr. 8 aufweist.

7. Immunogener Komplex des Typs, welcher ein immunogenes Element assoziiert mit einem Adjuvans, welches die Intensität der Immunantwort verstärkt, umfasst, **dadurch gekennzeichnet, dass** das immunogene Element ein Antigen oder ein Hapten ist und das Adjuvans ein Erzeugnis nach einem der Ansprüche 1 bis 6 umfasst.

8. Immunogener Komplex nach Anspruch 7, **dadurch gekennzeichnet, dass** das immunogene Element mit dem Adjuvans durch eine kovalente Bindung verbunden ist.

9. Immunogener Komplex nach einem der Ansprüche 7 oder 8, **dadurch gekennzeichnet, dass** das immunogene Element aus einem Fragment des Proteins G des RSV gebildet wird.

10. Immunogener Komplex nach einem der Ansprüche 7 bis 9, **dadurch gekennzeichnet, dass** das immunogene Element, welches mit dem Adjuvans assoziiert ist, mit einem Protein fusioniert ist, welches ein Rezeptor für ein Serumprotein, insbesondere für das menschliche Serumalbumin ist.

11. In vitro-Verfahren zur Erhöhung der Immunogenität eines Antigens oder eines Haptens, **dadurch gekennzeichnet, dass** man das Antigen oder Hapten mit einem Adjuvans nach einem der Ansprüche 1 bis 6 in Form eines Komplexes nach einem der Ansprüche 7 bis 10 kombiniert.

12. Verfahren nach Anspruch 11, **dadurch gekennzeichnet, dass** man das Antigen oder das Haptem mit dem Adjuvans durch chemische Kopplung verbindet.

13. Verfahren nach einem der Ansprüche 11 oder 12, **dadurch gekennzeichnet, dass** das Antigen oder Hapten durch Gentechnologie mit dem Adjuvans fusioniert ist.

14. Impfstoff, **dadurch gekennzeichnet, dass** er einen Komplex nach einem der Ansprüche 7 bis 10, welcher durch das Verfahren nach einem der Ansprüche 11 bis 13 hergestellt werden kann, enthält.

15. Verfahren zur Herstellung eines Adjuvans-Erzeugnisses, Proteins oder Peptids nach einem der Ansprüche 1 bis 6 ausgehend von Membranen von Bakterien der Gattung Klebsiella pneumoniae, **dadurch gekennzeichnet, dass** es die Schritte umfasst:
a) Ausfällung der Lipopolysaccharide durch Zugabe von Detergens und eines Salzes eines zweiwertigen Kations und Gewinnung des Überstands,
b) Ausfällung der Proteine des Überstands und Resuspendierung des Bodensatzes,
c) Chromatographie der Suspension mittels eines Anionenaustauschers und Gewinnung der Fraktionen, welche das Adjuvans-Erzeugnis enthalten,
d) Chromatographie mittels eines Kationenaustauschers und Gewinnung der Fraktion, welche das Adjuvans-Erzeugnis enthält,
e) Aufkonzentrierung der am Ende von Schritt d) erhaltenen Fraktion, um ein Adjuvans-Erzeugnis in Form von Protein, welches im wesentlichen frei von Lipopolysacchariden ist, zu gewinnen.


### LISTE DE SEQUENCES

(1) INFORMATION GENERALE:
   (i) DEPOSANT:
      (A) NOM: PIERRE FABRE MEDICAMENTS
      (B) RUE: 45, PLACE ABEL GANCE
      (C) VILLE: BOULOGNE
      (E) PAYS: FRANCE
      (F) CODE POSTAL: 92654
   (ii) TITRE DE L' INVENTION: PROTEINE P40
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1008 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE ADDITIONELLE:
      (A) NOM/CLE: CDS
      (B) EMPLACEMENT: 1..1008
   (xi) DESCRIPTION DE LA SEQUENCE: SEQ ID NO: 1:
(2) INFORMATION POUR LA SEQ ID NO: 2:
   (i) CARACTERISTIQUES DE LA SEQUENCE:
      (A) LONGUEUR: 335 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Ala Pro Lys Asp Asn Thr Trp Tyr Ala Gly Gly Lys Leu Gly Trp Ser
    1               5                  10                  15
   Gln Tyr His Asp Thr Gly Phe Tyr Gly Asn Gly Phe Gln Asn Asn Asn
               20                  25                  30
   Gly Pro Thr Arg Asn Asp Gln Leu Gly Ala Gly Ala Phe Gly Gly Tyr
           35                  40                  45
   Gln Val Asn Pro Tyr Leu Gly Phe Glu Met Gly Tyr Asp Trp Leu Gly
       50                  55                  60
   Arg Met Ala Tyr Lys Gly Ser Val Asp Asn Gly Ala Phe Lys Ala Gln
   65                  70                  75                  80
   Gly Val Gln Leu Thr Ala Lys Leu Gly Tyr Pro Ile Thr Asp Asp Leu
                   85                  90                  95
   Asp Ile Tyr Thr Arg Leu Gly Gly Met Val Trp Arg Ala Asp Ser Lys
               100                 105                 110
   Gly Asn Tyr Ala Ser Thr Gly Val Ser Arg Ser Glu His Asp Thr Gly
           115                 120                 125
   Val Ser Pro Val Phe Ala Gly Gly Val Glu Trp Ala Val Thr Arg Asp
       130                 135                 140
   Ile Ala Thr Arg Leu Glu Tyr Gln Trp Val Asn Asn Ile Gly Asp Ala
   145                 150                 155                 160
   Gly Thr Val Gly Thr Arg Pro Asp Asn Gly Met Leu Ser Leu Gly Val
                   165                 170                 175
   Ser Tyr Arg Phe Gly Gln Glu Asp Ala Ala Pro Val Val Ala Pro Ala
               180                 185                 190
   Pro Ala Pro Ala Pro Glu Val Ala Thr Lys His Phe Thr Leu Lys Ser
           195                 200                 205
   Asp Val Leu Phe Asn Phe Asn Lys Ala Thr Leu Lys Pro Glu Gly Gln
       210                 215                 220
   Gln Ala Leu Asp Gln Leu Tyr Thr Gln Leu Ser Asn Met Asp Pro Lys
   225                 230                 235                 240
   Asp Gly Ser Ala Val Val Leu Gly Tyr Thr Asp Arg Ile Gly Ser Glu
                   245                 250                 255
   Ala Tyr Asn Gln Gln Leu Ser Glu Lys Arg Ala Gln Ser Val Val Asp
               260                 265                 270
   Tyr Leu Val Ala Lys Gly Ile Pro Ala Gly Lys Ile Ser Ala Arg Gly
           275                 280                 285
   Met Gly Glu Ser Asn Pro Val Thr Gly Asn Thr Cys Asp Asn Val Lys
       290                 295                 300
   Ala Arg Ala Ala Leu Ile Asp Cys Leu Ala Pro Asp Arg Arg Val Glu
   305                 310                 315                 320
   Ile Glu Val Lys Gly Tyr Lys Glu Val Val Thr Gln Pro Ala Gly
                   325                 330                 335
(2) INFORMATION POUR LA SEQ ID NO: 3:
   (i) CARACTERISTIQUES DE LA SEQUENCE:
      (A) LONGUEUR: 537 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE ADDITIONELLE:
      (A) NOM/CLE: CDS
      (B) EMPLACEMENT: 1..537
   (xi) DESCRIPTION DE LA SEQUENCE: SEQ ID NO: 3:
   GCT CCG AAA GAT AAC ACC TGG TAT GCA GGT GGT AAA CTG GGT TGG TCC      48
   Ala Pro Lys Asp Asn Thr Trp Tyr Ala Gly Gly Lys Leu Gly Trp Ser
     1             5                  10                  15
   CAG TAT CAC GAC ACC GGT TTC TAC GGT AAC GGT TTC CAG AAC AAC AAC      96
   Gln Tyr His Asp Thr Gly Phe Tyr Gly Asn Gly Phe Gln Asn Asn Asn
                20                  25                  30
   GGT CCG ACC CGT AAC GAT CAG CTT GGT GCT GGT GCG TTC GGT GGT TAC     144
   Gly Pro Thr Arg Asn Asp Gln Leu Gly Ala Gly Ala Phe Gly Gly Tyr
            35                  40                  45
   CAG GTT AAC CCG TAC CTC GGT TTC GAA ATG GGT TAT GAC TGG CTG GGC     192
   Gln Val Asn Pro Tyr Leu Gly Phe Glu Met Gly Tyr Asp Trp Leu Gly
        50                  55                  60
   CGT ATG GCA TAT AAA GGC AGC GTT GAC AAC GGT GCT TTC AAA GCT CAG     240
   Arg Met Ala Tyr Lys Gly Ser Val Asp Asn Gly Ala Phe Lys Ala Gln
    65                  70                  75                  80
   GGC GTT CAG CTG ACC GCT AAA CTG GGT TAC CCG ATC ACT GAC GAT CTG     288
   Gly Val Gln Leu Thr Ala Lys Leu Gly Tyr Pro Ile Thr Asp Asp Leu
                    85                  90                  95
   GAC ATC TAC ACC CGT CTG GGC GGC ATG GTT TGG CGC GCT GAC TCC AAA     336
   Asp Ile Tyr Thr Arg Leu Gly Gly Met Val Trp Arg Ala Asp Ser Lys
               100                 105                 110
   GGC AAC TAC GCT TCT ACC GGC GTT TCC CGT AGC GAA CAC GAC ACT GGC     384
   Gly Asn Tyr Ala Ser Thr Gly Val Ser Arg Ser Glu His Asp Thr Gly
           115                 120                 125
   GTT TCC CCA GTA TTT GCT GGC GGC GTA GAG TGG GCT GTT ACT CGT GAC     432
   Val Ser Pro Val Phe Ala Gly Gly Val Glu Trp Ala Val Thr Arg Asp
       130                 135                 140
   ATC GCT ACC CGT CTG GAA TAC CAG TGG GTT AAC AAC ATC GGC GAC GCG     480
   Ile Ala Thr Arg Leu Glu Tyr Gln Trp Val Asn Asn Ile Gly Asp Ala
   145                 150                 155                 160
   GGC ACT GTG GGT ACC CGT CCT GAT AAC GGC ATG CTG AGC CTG GGC GTT     528
   Gly Thr Val Gly Thr Arg Pro Asp Asn Gly Met Leu Ser Leu Gly Val
                   165                 170                 175
   TCC TAC CGC                                                         537
   Ser Tyr Arg
(2) INFORMATION POUR LA SEQ ID NO: 4:
   (i) CARACTERISTIQUES DE LA SEQUENCE:
      (A) LONGUEUR: 179 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Ala Pro Lys Asp Asn Thr Trp Tyr Ala Gly Gly Lys Leu Gly Trp Ser
    1               5                  10                  15
   Gln Tyr His Asp Thr Gly Phe Tyr Gly Asn Gly Phe Gln Asn Asn Asn
               20                  25                  30
   Gly Pro Thr Arg Asn Asp Gln Leu Gly Ala Gly Ala Phe Gly Gly Tyr
           35                  40                  45
   Gln Val Asn Pro Tyr Leu Gly Phe Glu Met Gly Tyr Asp Trp Leu Gly
       50                  55                  60
   Arg Met Ala Tyr Lys Gly Ser Val Asp Asn Gly Ala Phe Lys Ala Gln
   65                  70                  75                  80
   Gly Val Gln Leu Thr Ala Lys Leu Gly Tyr Pro Ile Thr Asp Asp Leu
                   85                  90                  95
   Asp Ile Tyr Thr Arg Leu Gly Gly Met Val Trp Arg Ala Asp Ser Lys
               100                 105                 110
   Gly Asn Tyr Ala Ser Thr Gly Val Ser Arg Ser Glu His Asp Thr Gly
           115                 120                 125
   Val Ser Pro Val Phe Ala Gly Gly Val Glu Trp Ala Val Thr Arg Asp
       130                 135                 140
   Ile Ala Thr Arg Leu Glu Tyr Gln Trp Val Asn Asn Ile Gly Asp Ala
   145                 150                 155                 160
   Gly Thr Val Gly Thr Arg Pro Asp Asn Gly Met Leu Ser Leu Gly Val
                   165                 170                 175
   Ser Tyr Arg
(2) INFORMATION POUR LA SEQ ID NO: 5:
   (i) CARACTERISTIQUES DE LA SEQUENCE:
      (A) LONGUEUR: 216 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE ADDITIONELLE:
      (A) NOM/CLE: CDS
      (B) EMPLACEMENT: 1..216
   (xi) DESCRIPTION DE LA SEQUENCE: SEQ ID NO: 5:
   CGC GCT GAC TCC AAA GGC AAC TAC GCT TCT ACC GGC GTT TCC CGT AGC        48
   Arg Ala Asp Ser Lys Gly Asn Tyr Ala Ser Thr Gly Val Ser Arg Ser
    1               5                  10                  15
   GAA CAC GAC ACT GGC GTT TCC CCA GTA TTT GCT GGC GGC GTA GAG TGG        96
   Glu His Asp Thr Gly Val Ser Pro Val Phe Ala Gly Gly Val Glu Trp
               20                  25                  30
   GCT GTT ACT CGT GAC ATC GCT ACC CGT CTG GAA TAC CAG TGG GTT AAC       144
   Ala Val Thr Arg Asp Ile Ala Thr Arg Leu Glu Tyr Gln Trp Val Asn
           35                  40                  45
   AAC ATC GGC GAC GCG GGC ACT GTG GGT ACC CGT CCT GAT AAC GGC ATG       192
   Asn Ile Gly Asp Ala Gly Thr Val Gly Thr Arg Pro Asp Asn Gly Met
       50                  55                  60
   CTG AGC CTG GGC GTT TCC TAC CGC                                       216
   Leu Ser Leu Gly Val Ser Tyr Arg
    65                  70
(2) INFORMATION POUR LA SEQ ID NO: 6:
   (i) CARACTERISTIQUES DE LA SEQUENCE:
      (A) LONGUEUR: 72 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Arg Ala Asp Ser Lys Gly Asn Tyr Ala Ser Thr Gly Val Ser Arg Ser
    1               5                  10                  15
   Glu His Asp Thr Gly Val Ser Pro Val Phe Ala Gly Gly Val Glu Trp
               20                  25                  30
   Ala Val Thr Arg Asp Ile Ala Thr Arg Leu Glu Tyr Gln Trp Val Asn
           35                  40                  45
   Asn Ile Gly Asp Ala Gly Thr Val Gly Thr Arg Pro Asp Asn Gly Met
       50                  55                  60
   Leu Ser Leu Gly Val Ser Tyr Arg
    65                  70
(2) INFORMATION POUR LA SEQ ID NO: 7:
   (i) CARACTERISTIQUES DE LA SEQUENCE:
      (A) LONGUEUR: 159 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE ADDITIONELLE:
      (A) NOM/CLE: CDS
      (B) EMPLACEMENT: 1..159
   (xi) DESCRIPTION DE LA SEQUENCE: SEQ ID NO: 7:
   ACT GGC GTT TCC CCA GTA TTT GCT GGC GGC GTA GAG TGG GCT GTT ACT          48
   Thr Gly Val Ser Pro Val Phe Ala Gly Gly Val Glu Trp Ala Val Thr
     1               5                  10                  15
   CGT GAC ATC GCT ACC CGT CTG GAA TAC CAG TGG GTT AAC AAC ATC GGC          96
   Arg Asp Ile Ala Thr Arg Leu Glu Tyr Gln Trp Val Asn Asn Ile Gly
                20                  25                  30
   GAC GCG GGC ACT GTG GGT ACC CGT CCT GAT AAC GGC ATG CTG AGC CTG         144
   Asp Ala Gly Thr Val Gly Thr Arg Pro Asp Asn Gly Met Leu Ser Leu
            35                  40                  45
   GGC GTT TCC TAC CGC                                                     159
   Gly Val Ser Tyr Arg
        50
(2) INFORMATION POUR LA SEQ ID NO: 8:
   (i) CARACTERISTIQUES DE LA SEQUENCE:
      (A) LONGUEUR: 53 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Thr Gly Val Ser Pro Val Phe Ala Gly Gly Val Glu Trp Ala Val Thr
     1               5                  10                  15
   Arg Asp Ile Ala Thr Arg Leu Glu Tyr Gln Trp Val Asn Asn Ile Gly
                20                  25                  30
   Asp Ala Gly Thr Val Gly Thr Arg Pro Asp Asn Gly Met Leu Ser Leu
            35                  40                  45
   Gly Val Ser Tyr Arg
        50